# EUROPÄISCHE PATENTANMELDUNG

(11) **EP 2 684 587 A1**
(43) Veröffentlichungstag der Anmeldung: **15.01.2014**
(21) Anmeldenummer: 13001771.8
(22) Anmeldetag: 06.04.2013
(51) Int. Cl.: B01D 29/11

(54) **Vorrichtung und Verfahren zur Zellseparation**

(30) Priorität: 09.05.2012 DE 102012009088
(71) Anmelder: Forschungszentrum Jülich GmbH, 52425 Jülich (DE)
(72) Erfinder: Paczia, Nicole, 40468 Düsseldorf (DE); Noack, Stephan, 41844 Wegberg (DE); Schramm, Christian, 52428 Jülich (DE)

(57) **Zusammenfassung**

Die Erfindung betrifft eine Vorrichtung und ein Verfahren zur Zellseparation. Erfindungsgemäß wird eine Flüssigkeit mit Kühlmittel, enthaltend Zellen aus einem Bioreaktor über ein Filtermodul (1), bei dem ein Filter (7) über einer Fritte (3) befestigt ist, in eine Fraktion aus Flüssigkeit und Zellen getrennt. Bei der Weiterverarbeitung der so gewonnenen Zellen wird das gesamte Filtermodul (1) mit den Zellen einem Gefäß zugeführt, in dem die Zellen nach einem Omics-Verfahren weiter behandelt werden können.

## Beschreibung

Die Erfindung betrifft eine Vorrichtung und ein Verfahren zur Zellseparation.

In der Biotechnologie werden Mikroorganismen und deren Stoffwechselleistungen genutzt. Um die Ausbeute von biotechnologischen Produktionsprozessen zu steigern, können Prozessfaktoren, wie beispielsweise die Zusammensetzung von Nährmedien, entweder zufällig, d.h. durch Versuch und Irrtum, oder systematisch durch zielgerichtete Optimierung verbessert werden. Das setzt allerdings ein fundamentales Wissen über alle relevanten Systemkomponenten voraus und bedingt damit gleichzeitig eine ganzheitliche Beschreibung aller zellulären Funktionen des eingesetzten biologischen Systems, bzw. der eingesetzten Mikroorganismen. Die Untersuchung der Mikroorganismen kann dabei durch quantitative Omics-Analyse, wie beispielsweise die Metabolom-Analyse erfolgen, bei der Substrate, Produkte und Intermediate des mikrobiellen Stoffwechsels untersucht werden. Die Metabolom-Analyse wird in der Veröffentlichung "Metabolomics: current state and evolving methodologies and tools" von Oldiges et.al. in der Zeitschrift Applied Microbiology and Biotechnology 76: 495-511 beschrieben. Nach dieser Methode wird biologisches Material aus einem Kultivierungssystem entnommen, der Stoffwechsel des biologischen Materials muss - beispielsweise durch die Verringerung der Umgebungstemperatur - deaktiviert und die Zellen müssen von der umgebenden Flüssigkeit getrennt werden. Zur Verringerung der Umgebungstemperatur wird die Reaktorflüssigkeit, welche die Zellen enthält, in ein Kühlmittel eingeleitet, welches die biochemischen Abläufe in der Zelle quencht, so dass der aktuelle Stoffwechselzustand von Zellen im Reaktor konserviert wird. Die intrazellulären Metabolite der Zelle müssen aus den Zellen extrahiert und anschließend analysiert werden.
Der Schritt der Zellseparation hat unabhängig davon ob die durchgeführte Analyse dem Metabolom oder anderen Zellkomponenten, wie Proteinen, Metaboliten, Substraten, Produkten, Membranbestandteilen oder Intermediaten gilt, einen großen Einfluss.
Der Verfahrensschritt der Zellseparation sollte optimalerweise vollständig erfolgen, wobei die für die Separation benötigte Zeit so kurz und die auf die Zellen der Mikroorganismen wirkende Belastung so gering wie möglich gehalten werden sollte.

Nach dem Stand der Technik sind verschiedene Verfahren zur Zellseparation bekannt.

Ein klassisches Verfahren zur Zellabtrennung ist die Zentrifugation. Bei diesem Verfahren wird die Probe mit inaktivierten Zellen in ein Zentrifugationsröhrchen von standardisierter Größe, beispielsweise 15 ml oder 50 ml, gegeben und in einer kühlbaren Zentrifuge für 5 bis 15 Minuten auf bis zu 13000 g beschleunigt. Im Anschluss wird der zellfreie Überstand der Probe dekantiert und die weitere Bearbeitung der Mikroorganismen, insbesondere die Extraktion der Metabolite, kann direkt im Zentrifugenröhrchen erfolgen. Ein solches Verfahren ist in der Veröffentlichung von Wim de Koning et.al. unter dem Titel "A Method for Determination of Changes of Glycolytic Metabolites in Yeast on a Subsecond Time Scale Using Extraction at Neutral pH" in der Zeitschrift Analytical Biochemistry 204, 118-123 aus dem Jahr 1992 beschrieben.
Ein anderes Verfahren wird in der Veröffentlichung von Rutger D. et.al. in der Zeitschrift Biotechnology and Bioengeneering, Vol. 107, No. 1, Sept. 1, 2010 unter dem Titel "Intracellular Metabolite Determination in the Presence of Extracellular Abundance: Application to the Penicillin Biosynthesis Pathway in Penicillium chrysogenum" offenbart. Bei Verfahren dieser Art werden in Abhängigkeit vom untersuchten Organismus unterschiedliche Filter mit verschiedenen Porendurchmessern, Glasfilter oder Nylonmembranen verwendet. Für die Filtration werden die geeigneten Filter manuell auf einer porösen Trägerfläche, einer Fritte, platziert. Über einen Adapter wird mit Hilfe einer Pumpe ein Vakuum im Filtratraum erzeugt und die gekühlte Probe mit den inaktivierten Zellen auf den Filter gegeben. Nach der Filtration wird der mit den Zellen beladene Filter mit einer Pinzette von der porösen Trägerfläche genommen und in ein Gefäß überführt, welches sich für die weitere Bearbeitung eignet.

Bei der Zellseparation mittels Zentrifugation ist die Vollständigkeit der Zellabtrennung von den wirkenden g-Kräften und der Zentrifugationsdauer abhängig. Vollständigkeit sowie Schnelligkeit und niedriger Energieeintrag schließen sich bei der Zellseparation gegenseitig aus. Ein weiteres Problem der Zentrifugation ist das Probenvolumen, das durch die standardisierten Zentrifugationsgefäße vorgegeben ist. Das extrazelluläre Restvolumen ist mit 50 µl bis 300 µl sehr groß und weitgehend unabhängig von der untersuchten Menge an Biomasse. Das Flüssigkeitsvolumen wird dabei hauptsächlich von Flüssigkeitstropfen gebildet, die an den Wänden der Zentrifugationsgefäße haften und durch das Dekantieren nicht entfernt werden können. Das Waschen von Zellen ist nur bedingt möglich. Da die Zellen nach dem Zentrifugieren stark komprimiert am Boden des Zentrifugationsgefäßes haften, müssen sie während des Waschens durch starkes Schütteln resuspendiert werden. Nach der Resuspendierung müssen die Zellen durch einen weiteren Zentrifugationsschritt von der Waschlösung getrennt werden. Sowohl der Lösungsvorgang als auch die zweite Zentrifugation stellen einen weiteren Energieeintrag dar, der zum Verlust von Metaboliten oder anderen Komponenten als zu untersuchende Parameter führen kann. Darüber hinaus ist die Zentrifugation ein diskontinuierliches Verfahren, das nicht automatisiert werden kann. Weiterhin besteht das Problem, dass der Überstand bei der Zentrifugation nicht vollständig von den Zellen entfernt werden kann, so dass die nicht entfernte Überstandsflüssigkeit die weiteren Untersuchungen beeinflusst. Eine Veränderung der Zellen durch die verbliebene Überstandsflüssigkeit ist möglich. Soll eine Analyse intrazellulärer Bestandteile erfolgen, ist diese von der Überstandsflüssigkeit in besonderem Maß betroffen, da im Anschluss an die Zellseparation eine Extraktion dieser Bestandteile aus den Zellen erfolgt. Verbliebene Überstandsflüssigkeit verfälscht die Konzentration der Extrakte systematisch, weil sie einerseits das Extraktionsvolumen erweitert durch Verdünnung, und andererseits Stoffe aus der Überstandsflüssigkeit fälschlich dem Extrakt zugerechnet werden.

Die Filtration erfordert im Vergleich zur Zentrifugation einen höheren manuellen Einsatz, da die Kombination von Filtermodulen und geeigneten Filtern manuell erfolgt. Zudem müssen die Filter nach der Beladung mit Biomasse sorgfältig behandelt werden. Der Transfer der mit Biomasse beladenen Membranen in ein zur Weiterbearbeitung geeignetes Gefäß besitzt ein sehr hohes Fehlerpotential, da Zellverlust auftreten kann, und macht die Qualität der Ergebnisse stark abhängig vom manuellen Geschick des Experimentators. Da die Inaktivierung der Zellen meist durch den Einsatz von kalten organischen Lösungsmitteln erfolgt besteht die Gefahr von toxischen Einwirkungen auf den Experimentator. Insbesondere wegen des langen Zeitaufwandes und der Ungenauigkeit bei der Verfahrensweise wird die Filtration für die Zellseparation nur selten eingesetzt.

Beiden Verfahren ist gemein, dass der Stoffwechsel der Zellen durch ein Kühlmittel gestoppt wird, so dass ein Zellzustand konserviert wird, wie er dem Zustand der Zellen in dem Bioreaktor entspricht. Die Separation erfolgt dann jedenfalls in dem Kühlmittel. Während der Separation stehen die Zellen mit dem Kühlmittel in Kontakt, welches Einfluss auf die Zellen hat, und weiterhin bestehen während der Separation chemische, thermische und mechanische Einflüsse, die sich nachteilig auf die Zellen auswirken können. Diese Auswirkungen können dazu führen, dass die Zellparameter, die bestimmt werden sollen um einen Einblick in die Zellchemie im Reaktor zu erlangen, verändert werden können, wodurch es zu unerwünschten Verfälschungen der Messergebnisse kommen kann. Weiterhin besteht ein Zusammenhang zwischen der zellchemischen Zusammensetzung und der Zusammensetzung des Reaktorflüssigkeits-Kühlmittelgemischs. Daher ist es wichtig, dass sowohl die Zellen, als auch das Reaktorflüssigkeits-Kühlmittelgemisch bei der Separation vollständig und unverändert erhalten werden können. Je länger die einzelnen Verfahrensschritte dauern, desto größer ist der Wärmeeinfall in die zu untersuchenden Zellen.

Es ist daher die Aufgabe der Erfindung, ein Verfahren und eine Vorrichtung zur Zellseparation zur Verfügung zu stellen, mit denen die Separation von Zellen und Flüssigkeit vollständig erfolgt. Die Filtration soll experimentell sicher werden. Toxische Einflüsse für den Experimentator sollen minimiert oder verhindert werden und Materialverluste, insbesondere bei den Zellen, aber auch bei dem Reaktorflüssigkeits-Kühlmittelgemisch, sollen verhindert oder minimiert werden. Die zur Separation benötigte Zeit soll minimiert werden. Dabei soll der Verlust von zu untersuchenden Komponenten, wie beispielsweise Metabolite, Zellen, Substrat, Proteine, Membranbestandteile und Produkte verringert werden und eine zeitlich durchgehende Kühlung der Zellen gewährleistet sein. Die Belastung der Zellen, insbesondere durch chemische, thermische und mechanische Einflüsse, soll minimiert werden, um die Zellen so schonend wie möglich zu behandeln. Die Zellen, aber auch das abgetrennte Reaktorflüssigkeits-Kühlmittelgemisch, sollen bei dem Separationsprozess unverändert oder weitgehend unverändert bleiben. In der Omics-Analyse soll der Schritt der Separation optimiert werden und die erhöhte Arbeitsbelastung für den Experimentator und die damit verbundenen Fehlerquellen sollen verringert werden. Insgesamt soll der Einfluss der Umgebung auf die Zellen während der Separation so gering wie möglich gehalten werden, um systematische Fehler der Untersuchungsergebnisse zu vermeiden. Das Separationsverfahren soll automatisierbar sein. Von den Vorrichtungsbestandteilen sollen keine toxischen Einflüsse auf die Zellen ausgehen. Die Nachteile der Verfahren nach dem Stand der Technik sollen ausgeräumt werden.

Ausgehend vom Oberbegriff des Anspruchs 1 und des nebengeordneten Anspruchs wird die Aufgabe erfindungsgemäß gelöst mit den im kennzeichnenden Teil dieser Ansprüche angegebenen Merkmalen.

Mit dem erfindungsgemäßen Verfahren und der erfindungsgemäßen Vorrichtung ist es nunmehr möglich, eine Zellseparation vorzunehmen, die für die Zellen so schonend und schnell wie möglich ist und die eine vollständige Abtrennung der Zellen von dem Reaktor-Kühlflüssigkeitsgemisch ermöglicht, wobei die Einflüsse der Umgebung so gering wie möglich gehalten werden. Dadurch werden experimentelle Ergebnisse erzielt, die eine exaktere Bestimmung der Zellkomponenten und auch der Reaktorflüssigkeitskomponenten als nach dem Stand der Technik ermöglicht. Materialverluste können verhindert oder sehr stark begrenzt werden. Experimentelle Ungenauigkeiten durch Abweichungen der Handlung des Experimentators können verhindert werden und toxische Einflüsse auf den Experimentator können minimiert werden. Das Verfahren zur Zellseparation wird automatisierbar und die Größe der verwendeten Versuchsgefäße kann an die zur Untersuchung herangezogenen Probemengen angepasst werden. Die chemische Zusammensetzung der erfindungsgemäßen Vorrichtungsmaterialien wirkt sich nicht zelltoxisch aus.

Vorteilhafte Weiterbildungen der Erfindung sind in den Unteransprüchen angegeben.

Im Folgenden soll die Erfindung in ihrer allgemeinen Form beschrieben werden.

Die erfindungsgemäße Vorrichtung umfasst ein Filtermodul mit einer Fritte, einem Filter, beispielweise einer Membran, über die die Zellsuspension gefiltert werden kann und eine die Fritte und das Filter umgebende Wand. Erfindungsgemäß ist das Filter über der Fritte befestigt. Die Befestigung des Filters über der Fritte als Träger ist vorzugsweise nicht lösbar ausgestaltet. Besonders bevorzugt ist das Filtermodul mit der Fritte, der Wand und dem Filter einstückig ausgebildet. Insbesondere ist das gesamte Filtermodul einstückig ausgebildet.

Das Filtermodul kann unterschiedliche Geometrien aufweisen. Vorzugsweise besitzt es einen kreisförmigen Querschnitt. Es kann jedoch auch andere Geometrien aufweisen, beispielsweise oval oder eckig, beispielsweise quadratisch oder mehreckig.

Das Filtermodul weist eine Fritte auf, welche ein Abstützen einer Membran ermöglicht. Bei der Fritte kann es sich um ein Gitter, ein Sieb oder einen porösen Körper handeln, auf dem die Membran positioniert ist.

Die Wand des Filtermoduls kann an der Fritte enden oder sich über sie hinaus erstrecken. Der über die Fritte hinausragende Teil der Wand kann sich dabei gegenüber dem Querschnitt des Filtermoduls verjüngen oder mit gleichem Querschnitt weiter verlaufen. Vorzugsweise endet die Wand des Filtermoduls an der Fritte. Das hat den Vorteil, dass das über das Filtermodul abgezogene Reaktorflüssigkeits-Kühlmittelgemisch nicht an dem Filtermodul hängen bleibt und so vollständig gewonnen werden kann.

Im folgenden Teil der Beschreibung wird beispielhaft eine Membran als Filter eingesetzt. Die Beschreibung ist jedoch so auszulegen, dass an Stelle der Membran jedes geeignete Filter treten kann.
Erfindungsgemäß ist die Membran über der Fritte befestigt, so dass sie nicht aus dem Filtermodul herausfallen kann. Vorzugsweise ist die Membran nicht lösbar befestigt. Besonders bevorzugt ist die Wand des Filtermoduls mit der Fritte und der Membran einstückig ausgebildet. Als einstückig im Sinne der Erfindung ist sowohl eine lösbare Verbindung von Wand, Fritte und Membran, als auch eine nicht lösbare Verbindung zwischen diesen Teilen zu verstehen. Maßgeblich ist für die einstückige Ausführungsform, dass die Membran nicht aus dem Filtermodul mit der Fritte herausfallen oder herausgenommen werden kann, ohne dass Veränderungen an der Vorrichtung vorgenommen werden müssen oder dass mechanisch eingewirkt werden muss. Das Filter soll so befestigt sein, dass es bei dem Transfer des Filtermoduls zur weiteren Bearbeitung nicht herausfallen kann. Vorzugsweise ist das Filter in seinem Randbereich über der Fritte befestigt. Die Befestigung im Randbereich ist vorzugsweise durchgängig bzw. lückenlos. Die Wand des Filtermoduls bildet mit der Fritte einen offenen Hohlkörper, bei dem die Fritte den Boden bildet auf dem sich die Membran innenseitig befindet.

Die Befestigung der Membran kann unterschiedlich erfolgen. Beispielsweise kann die Membran an der Fritte und/oder an der Wand des Filtermoduls angeklebt oder angeschweißt sein. Sie kann auch mit Klemmmitteln über der Fritte befestigt sein, so dass sie nicht herausfällt oder herausgenommen werden kann.

Das Filter bzw. die Membran soll vorzugsweise dicht mit der Wand des Filtermoduls abschließen. Das hat den Vorteil, dass die Zellen bei der Filtration nicht an dem Filter bzw. der Membran vorbei in das Filtrat gelangen können.

Bei Betrieb wird die erfindungsgemäße Vorrichtung über einen Vakuumadapter auf ein Auffanggefäß für das Reaktorflüssigkeits-Kühlmittelgemisch mit Vakuumansatz gesetzt, über den das Reaktorflüssigkeits-Kühlmittelgemisch durch Anlegen eines Vakuums abgezogen und damit von den suspendierten Zellen separiert werden kann.

Die Befestigung der Membran an der Fritte und/oder der Wand des Filtermoduls führt dazu, dass der Experimentator das Filtermodul als Ganzes aus dem Adapter entnehmen und zur Weiterverarbeitung in eine Lösung überführen kann, in der dann die weiteren Verfahrensschritte vorgenommen werden können. Dadurch kann das Entnehmen einer losen Membran aus der Fritte entfallen.

Das hat zur Folge, dass ein reproduzierbarer Prozess ermöglicht wird, der Zellverluste vermeidet, da die gesamte Vorrichtung der Weiterverarbeitung zugeführt wird. Die Zellen werden geschont, da sie keinen mechanischen Belastungen ausgesetzt werden. Die Separation, insbesondere die Weiterverarbeitung, ist schneller als nach dem Stand der Technik und Fremdeinflüsse auf die Zelle werden vermieden, wodurch die bei der Weiterverarbeitung erhaltenen experimentellen Ergebnisse genauer sind. Materialverluste können vermieden werden. Toxische Einflüsse auf den Experimentator werden stark reduziert, da er nicht mehr mit dem Reaktorflüssigkeits-Kühlmittelgemisch in Berührung kommt. Experimentelle Ungenauigkeiten der Handlungsabweichungen des Experimentators können verhindert werden, da die Herausnahme der Membran entfällt und keine Zellverluste entstehen. Durch den Einsatz der erfindungsgemäßen Vorrichtung werden insgesamt bessere Ergebnisse der Omics-Analyse erzielt. Die erfindungsgemäße Vorrichtung kann als Einwegartikel verwendet werden; sie kann durch Gussverfahren leicht in jeder beliebigen Größe hergestellt werden, so dass die Größe der Vorrichtung an das gezogene Probenvolumen angepasst werden kann. Der Wegfall der Entnahme der Membran aus dem Filtermodul und die Weiterverarbeitung mittels der gesamten Vorrichtung hat zur Folge, dass das erfindungsgemäße Verfahren leicht automatisiert werden kann.

Die Befestigung der Membran an der Fritte und/oder der Wand des Filtermoduls kann unterschiedlich ausgestaltet sein. Die Möglichkeiten orientieren sich dabei an den verwendeten Materialien.

Ein Verschweißen ist insbesondere bei Metallen oder Kunststoffen möglich. Als verschweißbare Kunststoffe kommen insbesondere Polypropylen und Nylon in Betracht.

Eine Verklebung ist grundsätzlich bei allen Materialien möglich, jedoch darf das Klebemittel nicht in dem Reaktorflüssigkeits-Kühlmittelgemisch löslich und darf auch nicht zelltoxisch sein. Weiterhin soll das Klebemittel nicht in den eingesetzten Lösungsmitteln, insbesondere organischen Lösungsmitteln, der Reaktorflüssigkeit, dem Kühlmittel aber auch Extraktionsmittel und Verdünnungsmittel etc. löslich sein.

Weiterhin kann die Membran vorzugsweise über Klemmmittel über der Fritte befestigt sein. Das Klemmmittel hat vorzugsweise die gleiche Querschnittsgeometrie wie das Filtermodul. Hierfür kommt beispielsweise ein Klemmring in Betracht, der in die Wand des Filtermoduls hineingeschoben wird.

In einer besonders bevorzugten Ausführungsform hat der Klemmring einen Außendurchmesser, der dem Innendurchmesser der Wand des Filtermoduls entspricht, so dass er passschlüssig in das Filtermodul eingeführt werden kann und die Membran über der Fritte fixiert. Die Wand des Filtermoduls kann dann an mindestens einer Stelle eine Nut oder Aushöhlung aufweisen, die eine Aufnahme einer Feder des Klemmrings ermöglicht, die eine Fixierung des Klemmmittels ermöglicht.

Bei einem röhrenförmigen Filtermodul wird dann ein röhrenförmiger Klemmring eingeführt, dessen Außendurchmesser dem Innendurchmesser der Wand des Filtermoduls entspricht, so dass eine passschlüssige Verbindung zu Stande kommt. Die Wand des Filtermoduls hat dann vorzugsweise an mindestens einer Stelle eine Nut zur Aufnahme einer Nobbe bzw. einer Feder des Klemmrings, der in sie hineingreift. Damit wird der Klemmring in der Wand des Filtermoduls fixiert. Vorzugsweise verläuft die Nut entlang des Innendurchmessers der Wand des Filtermoduls kreisförmig und der Klemmring weist eine ringförmig verlaufende Erweiterung seines Außendurchmessers auf, der genau in die ringförmige Nut der Wand passt, so dass eine passschlüssige Verbindung zwischen dem Klemmring und der Wand zu Stande kommt. Es ist auch eine Ausführungsform möglich, in der der Klemmring eine Nut oder Aushöhlung aufweist, in die eine Nobbe oder Feder eingreifen kann, die sich an der Wand befindet. Es kann grundsätzlich jedes Nut-Federsystem eingesetzt werden, um den Klemmring bzw. die Klemmmittel zu fixieren.

Vorzugsweise befindet sich die Feder des Klemmrings in einer Position, die so weit von seinem eingeführtem Ende entfernt ist, wie die Nut des Filtermoduls von der Fritte, so dass die Membran beim Einrasten der Feder in die Nut fest an den Randbereich der Fritte gedrückt wird. Von dem Begriff des Klemmrings sollen auch ringförmige Klemmkörper umfasst sein, die eine andere Querschnittsgeometrie haben als einen Kreisquerschnitt, nämlich auch die oben genannten ovalen oder viereckigen bzw. mehreckigen Geometrien.

Vorteilhaft weist die Wand eine Führung auf, in die der Klemmring eingreifen kann und entlang der er in das Filtermodul eingeführt werden kann. Bei der Führung kann es sich um ein Feder-Nut System handeln, entlang dem der Klemmring eingeführt wird. Damit wird ein radiales Verrutschen des Klemmrings verhindert. Das ist insbesondere für eine Ausführungsform vorteilhaft, bei der die Fritte und die Membran gegenüber dem Querschnitt des Filtermoduls geneigt sind.

Weiterhin ist es vorteilhaft, wenn die Außenmaße des Klemmrings bezüglich seiner Länge genau die gleichen Abmessungen haben, wie die Wand des Filtermoduls. Dadurch werden Toträume im Innenraum vermieden.

Vorzugsweise ist das erfindungsgemäße Filtermodul aus Materialien gefertigt, die keine toxischen Einflüsse auf die Zellen haben. Die Wand, die Fritte und das Klemmmittel können aus Kunststoff oder Metall bestehen. Beispielhaft aber nicht beschränkend können als Materialien Polypropylen, Nylon, Polyethylen, Polyester, Polyamid oder Edelstahl genannt werden. Besonders beständig gegenüber organischen Lösungsmitteln sind Polypropylen und Nylon, weswegen diese Materialen besonders gut geeignet sind, so dass von ihnen keine zelltoxische Wirkung ausgeht. Dabei können die Materialien für unterschiedliche Vorrichtungsbestandteile verschieden sein, so dass auch Materialkombinationen möglich sind.

Die Wahl der Membran oder des Filters hängt von den zu untersuchenden Zellen ab.

Als Materialien sind Nylon, Polyamid, PTFE, Polyester, Zellulosenitrat und Glas, beispielsweise Glasfaser, besonders gut geeignet.

Die Porengrößen sollen so ausgewählt sein, dass sie insbesondere bei den Temperaturen des Kühlmittels bzw. der Flüssigkeit geeignet sind, Zellen zurückzuhalten. Typische Temperaturen liegen zwischen - 30° C und - 10° C, insbesondere bei - 20° C. Typische Porengrößenbereiche sind 0,2 µm bis 200 µm, jedoch ist diese Angabe nicht beschränkend.

Beispielhaft können 0,22 µm bis 2 µm für Bakterien, 1 µm bis 5 µm für Hefen und 5 um bis 100 µm für hyphig wachsende Pilze verwendet werden.

Dem Fachmann ist bekannt, welche Membranen für welche Zellen besonders gut geeignet sind.

Beispielhaft kann für die Untersuchung bakterieller Systeme eine Membran aus Polyamid mit einer Porenweite von 1,2 µm eingesetzt werden, während für die Untersuchung hyphig wachsender Pilze eine Nylonmembran mit einer Porenweite von 100 µm eingesetzt werden kann. Durch den modularen Aufbau der erfindungsgemäßen Vorrichtung ist es möglich, die Eigenschaften der Membran, also Material und Porenweite, den Anforderungen der gegebenen Untersuchung anzupassen.

Zwischen der Fritte und der Membran bzw. dem Filter kann Dichtmittel angebracht sein. Bei dem Dichtmittel kann es sich um eine weitere Schicht handeln, die zwischen dem Filter und der Fritte positioniert ist. Es kommt auch ein Dichtring in Betracht. Das Dichtmittel führt zur Dichtigkeit der Klemmung der Membran. Insbesondere bei Materialien für Filter oder Membranen, die selber keine gute Dichtung bewirken, weil sie beispielsweise zu spröde oder zu wenig flexibel sind, ist die Verwendung von Dichtmitteln vorteilhaft.

Um eine dichte Klemmung jeder verwendeten Membran zwischen Außenhülle und Klemmring zu garantieren, besteht beispielsweise die Möglichkeit, die Membran mit einer Abdichtschicht aus Glasfaser zu unterlegen. Der Faserabstand der Abdichtschicht wird dabei so gewählt, dass die Stützschicht selbst keinen Beitrag zur Filtration leistet und die Filtrationszeit nicht erhöht. Für die Untersuchung von Mikroorganismen ist z.B. die Kombination einer Polyamidmembran mit einer Porenweite von 1.2 µm und einer Glasfaserstützschicht mit 52 µm Faserabstand geeignet. Die Verwendung einer Stützschicht ist nicht bei jedem Membranmaterial erforderlich. Besonders bevorzugt ist eine Stützschicht für Membranen aus Polyamid.

Die Stützschicht kann beispielsweise aus Glasfaser bestehen.

Die Porengröße der Stützschicht ist bevorzugt das Zehnfache der Porengröße des Filters.

In einer Ausführungsform bildet die Fritte mit der Membran und ggf. als Dichtmittel fungierenden Stützschicht einen Boden des Filtermoduls, der zu der Längsachse des Filtermoduls senkrecht verläuft und damit einen bezüglich der Saugrichtung planen Boden ausbildet. Die Fritte mit der Membran befindet sich dabei im Querschnitt des Filtermoduls. Das hat den Vorteil, dass sich die gefilterten Zellen möglichst gleichmäßig über die gesamte Membranoberfläche verteilen.

In einer alternativen Ausführungsform ist die Filterfläche zum Querschnitt des Filtermoduls geneigt angeordnet, um eine größere Filterfläche zu erreichen, ohne dabei die Außenmaße zu vergrößern. Die Neigung der Membran beeinflusst die weitere Bearbeitung der Zellen nicht und dient der Vergrößerung der Filterfläche und bewirkt damit eine Verringerung der Filtrationszeit. Vorzugsweise ist die Fritte mit der Membran und ggf. der Stützschicht gegenüber dem Querschnitt des Filtermoduls um 30° bis 45° geneigt.

Die Geometrie und die Materialien der erfindungsgemäßen Vorrichtung sind so gewählt, dass sie die weitere Bearbeitung der Zellen nicht beeinflussen und eine Abtrennung der Zellen von der Filtrationseinheit daher nicht erforderlich ist. Das ermöglicht, dass das erfindungsgemäße Filtermodul mit den filtrierten Zellen als Ganzes der weiteren Bearbeitung zugeführt werden kann. Damit wird Zeit gespart und Ungenauigkeiten werden vermieden.

Bei dem erfindungsgemäßen Verfahren werden sich in einer Flüssigkeit befindende Zellen in das erfindungsgemäße Filtermodul gegeben und die flüssige Phase wird abgesaugt. Dabei kann ein Filtermodul eingesetzt werden, das jede beliebige Unterkombination von Vorrichtungsmerkmalen besitzt, die im Beschreibungsteil für die Vorrichtung offenbart ist. Bei der Flüssigkeit kann es sich beispielsweise um ein Reaktorflüssigkeits-Kühlmittelgemisch, die Reaktorflüssigkeit, ein organisches Lösungsmittel, Wasser oder eine andere Flüssigkeit handeln, in der sich die Zellen befinden. Erfindungsgemäß wird das Filtermodul nach dem Absaugen der flüssigen Phase mit den sich darin befindlichen Zellen in ein Gefäß gegeben, in dem ein weiterer Analyseschritt, beispielsweise eine gravimetrische Biomassebestimmung, eine mechanische Weiterverarbeitung, beispielsweise mit Ultraschall oder in einer Mühle als Beispiel für eine Zellaufschlussmethode oder eine Behandlung mit einer weiteren beispielsweise für die Omics-Analyse verwendeten Flüssigkeit als Analyse der Biomasse erfolgt. Das erfindungsgemäße Filtermodul kann daher in jeder Unterkombination seiner möglichen offenbarten Vorrichtungsmerkmale in der Omics-Analyse verwendet werden. Als Flüssigkeit kommt beispielsweise Methanol in Betracht. Dabei werden die sich in dem Filtermodul befindlichen Zellen mit einer Flüssigkeit in Kontakt gebracht, die auf sie einwirkt, ohne dass Zellverluste durch Herausnehmen eines Filters entstehen. Durch diesen Verfahrensschritt werden die Zellen nicht fremd kontaminiert, da sie nicht mit weiteren Stoffen in Kontakt kommen. Da das beladene Filter über der Fritte befestigt ist, gestaltet sich der Transfer sehr einfach, sicher und schnell. Die Gefahr, dass Biomasse verloren gehen könnte oder dass der Experimentator in Kontakt mit Lösungsmitteln kommt, besteht nicht. Die Eingangs beschriebenen Vorteile des erfindungsgemäßen Verfahrens werden verwirklicht.

### Spezieller Beschreibungsteil:

Im Folgenden wird die erfindungsgemäße Vorrichtung und das erfindungsgemäße Verfahren an Hand einer Ausführungsform beschrieben, ohne dass diese Beschreibung einschränkend auszulegen ist.

Die Figuren zeigen die erfindungsgemäße Vorrichtung.

Es zeigt.
- Fig. 1:: Eine erfindungsgemäße Vorrichtung
- Fig. 1a:: Einen Klemmring
- Fig. 2:: Einen Längsschnitt durch ein Filtermodul
- Fig. 2a:: Eine Detailansicht der Nut aus Figur 2
- Fig. 3:: Einen Querschnitt eines Klemmrings
- Fig. 3a:: Eine Detailansicht einer Feder, bzw. einer Feder des Klemmrings
- Fig. 4:: Eine Ansicht der erfindungsgemäßen Vorrichtung
- Fig. 5:: Einen Längsschnitt der erfindungsgemäßen Vorrichtung
- Fig. 5a:: Eine Detailansicht aus Figur 5
- Fig. 6a:: Einen Querschnitt der erfindungsgemäßen Vorrichtung mit einer Führung
- Fig. 6b:: Eine Detailansicht der Führung
- Fig. 6c:: Eine Aufsicht auf die erfindungsgemäße Vorrichtung mit Klemmring und Führung
- Fig. 6d:: Eine Aufsicht auf die erfindungsgemäße Vorrichtung mit Maßangaben
- Fig. 6e:: Eine Aufsicht auf die erfindungsgemäße Vorrichtung mit Maßangaben
- Fig. 6f:: Eine Aufsicht auf die erfindungsgemäße Vorrichtung mit Maßangaben
- Fig. 7a:: Einen Querschnitt eines Klemmrings mit einer Führung
- Fig. 7b:: Eine Detailansicht der Führung des Klemmrings
- Fig. 7c:: Einen Querschnitt des Klemmrings mit Führung

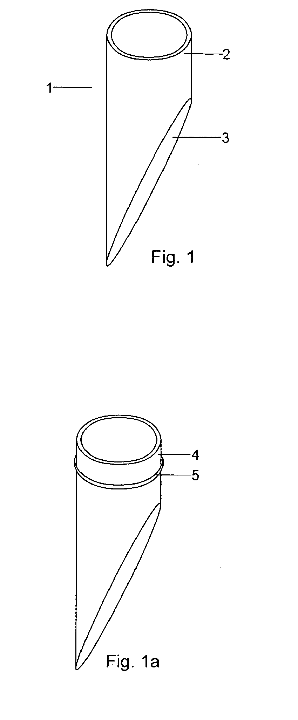

In Figur 1 ist die Außenansicht des erfindungsgemäßen Filtermoduls 1 dargestellt. Sie zeigt die Wand 2 des Filtermoduls 1 und die Fritte 3.

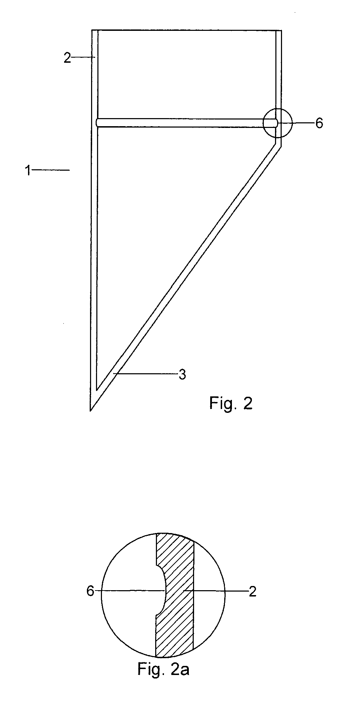

Figur 2 zeigt einen Klemmring 4 mit einer Feder 5.

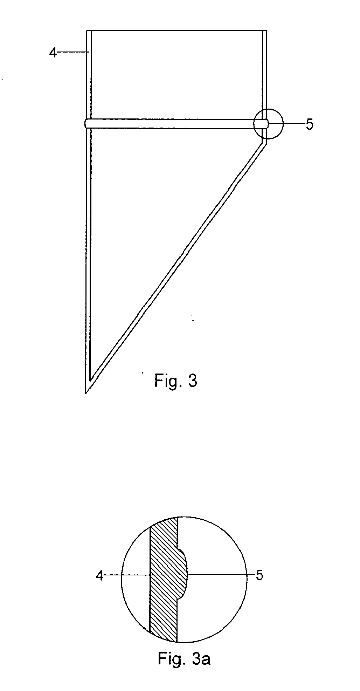

In Figur 3 haben gleiche Vorrichtungsmerkmale dieselben Bezugszeichen. In ihr ist eine sich über die röhrenförmige Wand 2 des Filtermoduls 1 verlaufende Nut 6 dargestellt.

Figur 2a zeigt eine Detailansicht der Nut 6 in der Wand 2.

Figur 3 zeigt den Klemmring 4 mit der um den Klemmring 4 ringförmig umlaufenden Feder 5.

Figur 3a bildet eine Detailansicht des Klemmrings 4 mit der Feder 5 ab.

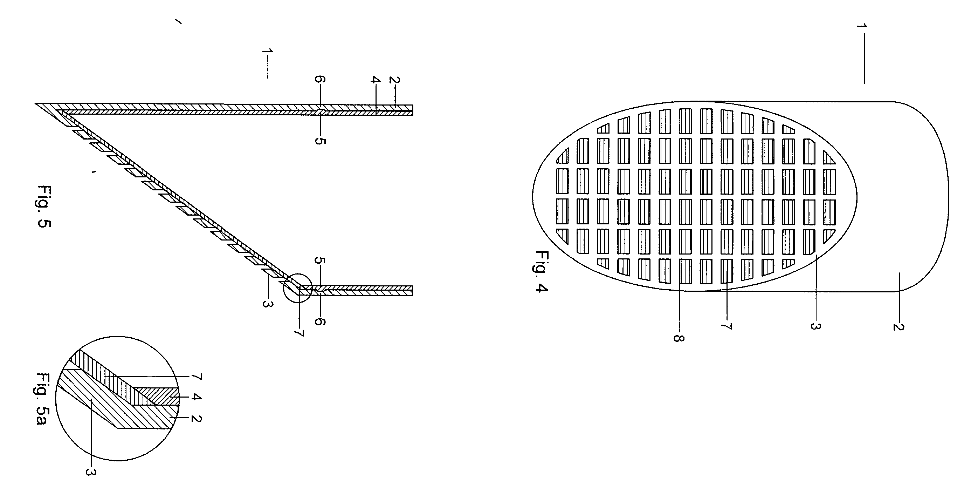

Figur 4 zeigt das Filtermodul 1 mit der Wand 2, der Fritte 3 und der Membran 7, die durch Öffnungen 8 der Fritte 3 sichtbar wird.

Figur 5 zeigt ein Filtermodul 1 mit einer Wand 2, in die ein Klemmring 4 aufgenommen ist. Die Wand 2 hat eine radial umlaufende Nut 6, in die die ebenfalls radial verlaufende Feder 5 des Klemmrings 4 eingreift und diesen fixiert. Der Klemmring 4 endet im unteren Bereich des Filtermoduls 1 auf der Membran 7 und hält diese auf der Fritte 3 fixiert, so dass die Membran 7 zwischen der Wand 2 und der Fritte 3 befestigt ist.

In Figur 5a ist ein Detailausschnitt der Figur 5 dargestellt, in dem die Membran 7 durch den Klemmring 4 in der Wand 2 und der Fritte 3 befestigt ist. In dieser Figur wird die Klemmung als Befestigungsmerkmal gut sichtbar.

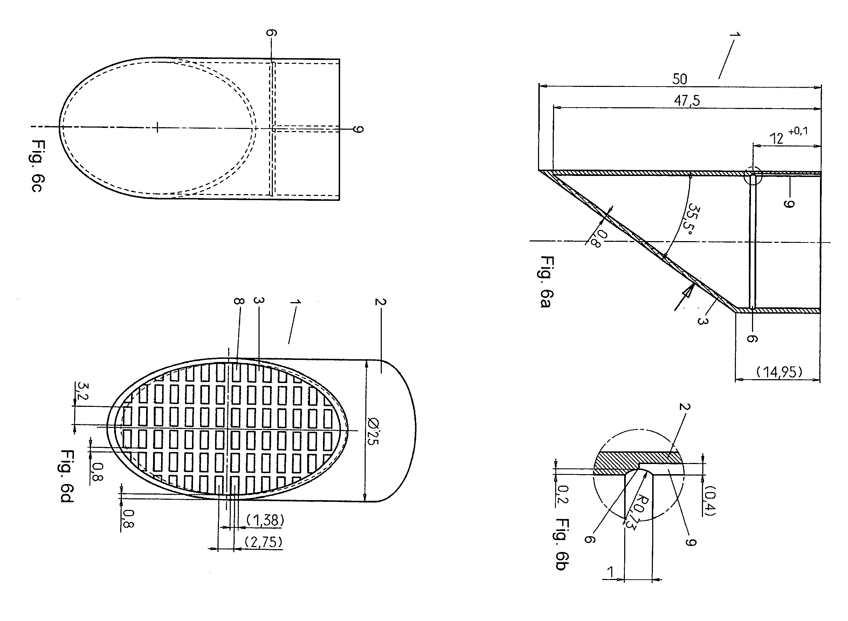

Figur 6a zeigt ein Filtermodul 1 mit einer Fritte 3 und einer Nut 6 im Querschnitt. An der Innenseite der Wand 2 ist eine Führung 9 angebracht, die in Form einer Nut in Längsachse zum Filtermodul 1 verläuft.

Figur 6b zeigt die Detailansicht der Führung 9 in der Wand 2. Die Führung 9 trifft auf die Nut 6 der Wand 2.

In Figur 6c ist eine Aufsicht des Filtrationsmoduls dargestellt, in der die Nut 6, und die Führung 9 schematisch dargestellt sind.

In Figur 6d haben die Vorrichtungsmerkmale gleiche Bezugszeichen, wie in den anderen Figuren.

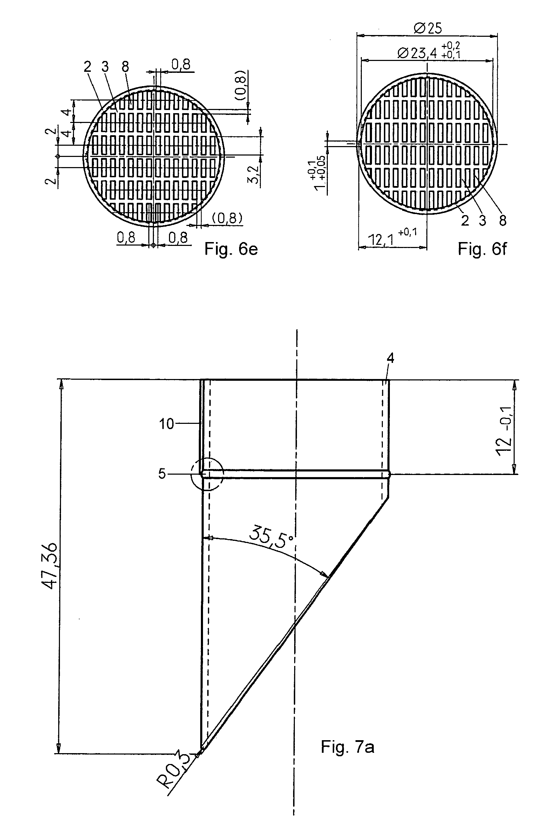

Die Figuren 6e und 6f zeigen eine Aufsicht auf die Fritte 3 mit den Öffnungen 8. Sie sind von der Wand 2 des Filtermoduls 1 umgeben.

Figur 7a zeigt einen Klemmring 4 mit einer Feder 5 und einer Führung 10 in Form einer Feder 10.

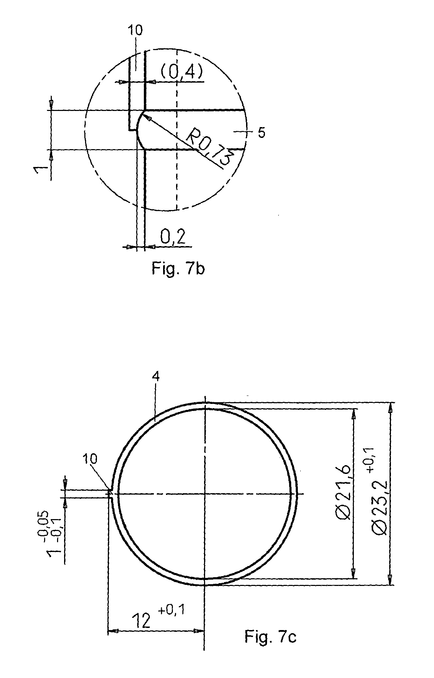

In Figur 7b ist eine Detailansicht der Führung 10 und der Feder 5 dargestellt.

Figur 7c zeigt einen Querschnitt durch den oberen Teil des Klemmrings 4 mit einer Feder als Führung 10.

## Patentansprüche

1. Filtermodul umfassend eine Fritte, ein Filter und eine das Filter und die Fritte umgebende Wand,
**dadurch gekennzeichnet,**
**dass** das Filter (7) über der Fritte (3) befestigt ist.

2. Filtermodul nach Anspruch 1,
**dadurch gekennzeichnet,**
**dass** es einstückig ausgebildet ist.

3. Filtermodul nach Anspruch 1 oder 2,
**dadurch gekennzeichnet,**
**dass** das Filter (7) eine Membran ist.

4. Filtermodul nach einem der Ansprüche 1 bis 3,
**dadurch gekennzeichnet,**
**dass** das Filter (7) eine Porengröße in einem Bereich zwischen 0,2 µm und 200 µm hat.

5. Filtermodul nach einem der Ansprüche 1 bis 4,
**dadurch gekennzeichnet,**
**dass** die Wand (2) und/oder die Fritte (3) aus Metall oder Kunststoff besteht.

6. Filtermodul nach Anspruch 5,
**dadurch gekennzeichnet,**
**dass** der Kunststoff eine Komponente aus der Gruppe Polypropylen, Nylon, Polyethylen, Polyester und Polyamid ist.

7. Filtermodul nach einem der Ansprüche 1 bis 6,
**dadurch gekennzeichnet,**
**dass** das Filter (7) aus Nylon, Polyamid, PTFE, Polyester, Zelluloseacetat, Glas oder Glasfaser besteht.

8. Filtermodul nach einem der Ansprüche 1 bis 7,
**dadurch gekennzeichnet,**
**dass** das Filter (7) durch Klemmmittel, Verkleben oder Verschweißen über der Fritte (3) befestigt ist.

9. Filtermodul nach Anspruch 8,
**dadurch gekennzeichnet,**
**dass** das Klemmmittel ein Klemmring (4) ist, dessen Außendurchmesser so groß ist, wie der Innendurchmesser der Wand (2), so dass eine passschlüssige Verbindung zu Stande kommt, wobei der Klemmring (4) das Filter (7) gegen die Fritte (3) fixiert.

10. Filtermodul nach Anspruch 8 oder 9,
**dadurch gekennzeichnet,**
**dass** das Klemmmittel oder die Wand (2) eine Nut (6) aufweisen, in die eine sich am Klemmmittel oder der Wand (2) befindliche Feder (5) eingreifen kann.

11. Filtermodul nach einem der Ansprüche 8 bis 10,
**dadurch gekennzeichnet,**
**dass** Führungsmittel (9,10) vorhanden sind, welche ein Einführen des Klemmmittels in die Wand (2) ermöglichen.

12. Filtermodul nach einem der Ansprüche 1 bis 11,
**dadurch gekennzeichnet,**
**dass** zwischen der Fritte (3) und dem Filter (7) ein Dichtmittel angebracht ist, welches eine dichte Verbindung des Filters (7) mit mindestens dem Randbereich der Fritte (3) ermöglicht.

13. Filtermodul nach einem der Ansprüche 1 bis 12,
**dadurch gekennzeichnet,**
**dass** die Fritte (3) und das Filter (7) zu dem Querschnitt des Filtermoduls (1), der senkrecht zur Längsrichtung des Filtermoduls (1) verläuft, geneigt ist.

14. Verwendung des Filtermoduls nach einem der Ansprüche 1 bis 13 in der Omics-Analyse.

15. Verfahren zur Zellseparation, bei dem sich in einer Flüssigkeit befindliche Zellen über
einen Filter eines Filtermoduls von Flüssigkeit abgesaugt werden und in einem folgenden Schritt einer Analyse der Biomasse zugeführt wird,
**dadurch gekennzeichnet,**
**dass** die Separation mit einem Filtermodul (1) nach einem der Ansprüche 1 bis 13 erfolgt und das Filtermodul, (1) welches die abgetrennten Zellen enthält, einem Gefäß zugeführt wird, in dem ein weiterer Schritt des Analyseverfahrens der Biomasse durchgeführt wird.
